(19) Europäisches Patentamt
European Patent Office
Office européen des brevets

(11) Publication number: 0 042 544 B1

(12) # EUROPEAN PATENT SPECIFICATION

(45) Date of publication of patent specification: 03.10.84

(21) Application number: 81104480.9

(22) Date of filing: 11.06.81

(51) Int. Cl.³: C 07 D 401/04, C 07 D 409/04, C 07 D 221/06, C 07 D 333/80, A 61 K 31/44, A 61 K 31/445

(54) **Novel antihistamines, process for their preparation and pharmaceutical compositions containing them.**

(30) Priority: 19.06.80 US 160795

(43) Date of publication of application: 30.12.81 Bulletin 81/52

(45) Publication of the grant of the patent: 03.10.84 Bulletin 84/40

(84) Designated Contracting States: AT BE CH DE FR GB IT LI LU NL SE

(56) References cited:
GB-A-1 360 219
US-A-3 264 342
US-A-3 326 924
US-A-3 435 073
US-A-3 464 983
US-A-3 491 103
US-A-3 513 201
US-A-3 763 169
US-A-3 786 095
US-A-3 917 669
US-A-3 952 017

HELVETICA CHIMICA ACTA, volume 49, no. 1, 1966 BASEL (CH) J.M. BASTIAN et al. "No. 26. 4-H-benzo (4,5) cyclohepta (1,2-b) thiophene", pages 214-34

(73) Proprietor: SCHERING CORPORATION
2000 Galloping Hill Road
Kenilworth, New Jersey 07033 (US)

(72) Inventor: Villani, Frank, J.
55 McKinley Avenue
West Caldwell New Jersey 07006 (US)

(74) Representative: Antony, Fritz, Dr. et al
P.O. Box 601 Winkelriedstrasse 35
CH-6002 Lucerne (CH)

EP 0 042 544 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel antihistamines, to processes for their preparation and to pharmaceutical compositions comprising the novel compounds.

United States Patent Nos. 3,464,983 and 3,491,103 and Helvetica Chimica Acta *49*, No. 1, 1966 pages 214—234 name various 4H - benzo[4,5]cyclohepta[1,2 - b]thiophenes and their 9,10-dihydro derivatives and 6-chloro derivatives having esterified carboxy aminoalkylidene or N-esterified carboxy piperidylidene groups attached at the 4-position, a typical compound being 4 - (3 - methyl - 3 - ethoxycarbonylaminopropylidene) - 9,10 - dihydro - 4H - benzo[4,5]cyclohepta[1,2 - b]thiophene. Such compounds are mentioned as intermediates in the production of derivatives in which the esterified carboxy function is replaced by hydrogen or alkyl, the resulting derivatives being antidepressants (3,464,983), or are antihistamines (3,491,103 and Helvetica Chimica Acta). United States Patent No. 3,326,924 describes various aza-dibenzo[a,b]cycloheptenes, and 9,10 derivatives, typically 4 - aza - 5 - (N - methyl - 4 - piperidylidene) - 10,11 - dihydro - 5H - dibenzo[a,d]cycloheptene; such compounds are characterized by their antihistamic, antiserotonin and antianaphylactic action.

United States Patents No. 3,264,342, 3,435,073, 3,513,201, 3,786,095, 3,917,669, 3,952,017 and 3,763,169 describe various 5H-dibenzo[a,d]cycloheptenes or 10,11-dihydro derivatives thereof bearing a side chain in the 5-position which is typically 3-N-methyl-N-carboethoxy aminopropyl. Such compounds are described as intermediates in the formation of derivatives in which for instance the carboethoxy function is replaced by hydrogen or an alkyl group, the derivatives being variously described as having gastric antisecretory properties (3,763,169 and 3,435,073), antidepressant properties (3,952,017, 3,786,095, 3,917,669, 3,513,201) and antihistaminic properties (3,264,342).

The compounds of this invention are of the general formula I

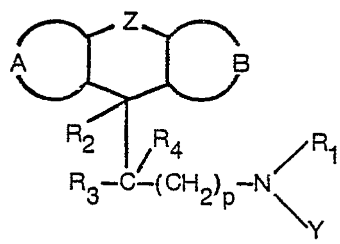

(I)

wherein

A represents the group of atoms required for completing a fused aromatic 5- or 6-membered heterocyclic ring or a fused benzene ring;

B represents the group of atoms required for completing a fused pyridine, benzene or X-substituted benzene ring wherein X is halogen, with the proviso that, when A defines a nitrogen-containing heterocyclic ring, then B defines a fused benzene or X-substituted benzene ring;

Z represents a single bond or a substituted or unsubstituted methylene or ethylene group, the substituent being an oxo or hydroxy group, or Z represents vinylene;

$R_2$ represents hydrogen or an alkyl group having 1 to 3 carbon atoms and

$R_3$ represents hydrogen, or

$R_2$ and $R_3$ form together a second bond;

$R_4$ represents hydrogen and

$R_1$ represents an alkyl group having 1 to 6 carbon atoms, or

$R_1$ and $R_4$ form together a second bridge $—(CH_2)_2—$ between the carbon atom and the nitrogen atom to which they are attached;

p represents 1 or 2; and

Y represents either of the groups $—COOR_7$ and $—SO_2R_8$

wherein

$R_7$ represents alkyl, cycloalkyl, cycloalkylalkyl, alkenyl, phenyl, phenylloweralkyl or 2-, 3- or 4-piperidyl and

$R_8$ represents alkyl, cycloalkyl, cycloalkylalkyl, alkenyl, phenyl or phenylloweralkyl, whereby the alkyl, cycloalkyl, cycloalkylalkyl and alkenyl groups represented by $R_7$ may optionally be substituted by the group

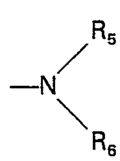

wherein $R_5$ and $R_6$ each represents hydrogen or an alkyl group having 1 to 6 carbon atoms, the piperidyl groups represented by $R_7$ may be substituted at the nitrogen atom by a lower alkyl group having 1 to 4 carbon atoms, and whereby the phenyl groups and the phenyl moiety of the phenylloweralkyl groups represented by $R_7$ and/or $R_8$ may be substituted by halogen or lower alkyl having 1 to 4 carbon atoms, subject to the following further provisos:

(i) that when A is a group of atoms required to form a fused [1,2-b] thiophene ring, Z is $-CH_2-CH_2-$ or $-CH=CH-$, p is 2 and either

1) B is a group of atoms required to form a fused benzene ring, $R_2$ and $R_3$ are both hydrogen or form a double bond, $R_4$ is hydrogen and $R_1$ is $C_1-C_4$ alkyl or $R_4$ and $R_1$ form the second bridge $(-CH_2-)_2$; or

2) B is an X substituted benzene ring, $R_2$ and $R_3$ form a double bond, $R_4$ is hydrogen and $R_1$ is Me; then $R_7$ cannot be $C_1-C_4$ alkyl or phenyl ($C_1-C_4$ alkyl); and

(ii) when A and B represent, respectively a fused benzene ring and a fused benzene or X substituted benzene ring then $R_1$ and $R_4$ form together a second bridge $(-CH_2-)_2$ between the carbon atom and nitrogen atom to which they are attached.

The alkyl and alkenyl groups in the above definitions of Y are such having up to 12 carbon atoms, most preferably up to 6 carbon atoms, and the loweralkyl groups (in the phenylloweralkyl groups) are such having 1 to 4 carbon atoms. The cycloalkyl groups have 3 to 7 and the cycloalkylalkyl groups 4 to 12 carbon atoms. All lower alkyl, alkyl and alkenyl groups referred to in the above definitions may be straight or branched chains.

In a preferred aspect Z represents unsubstituted or substituted ethylene, preferably unsubstituted ethylene, or vinylene.

Another preferred group of compounds are those wherein $R_2$ and $R_3$ together form a second bond.

The preferred definition of $R_1$ and $R_4$ is that $R_1$ and $R_4$ together form a second bridge of the formula $-(CH_2)_2-$.

Of the aromatic heterocyclic rings defined by A, the sulfur or nitrogen containing rings are preferred. Most preferably the heterocyclic ring is a thiophene or pyridine ring. In the thiophene ring the sulfur atom may be in either of the two possible positions and in the pyridine ring the nitrogen atom may be in any of the 4 possible positions.

The preferred definition of B is the one which provides a fused benzene or X-substituted benzene ring.

A most preferred group of compounds may be defined by the following formula II

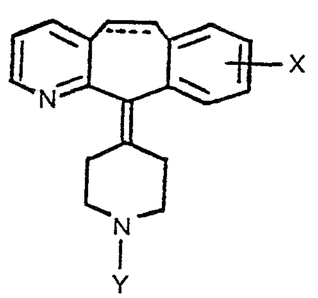

(II)

wherein the dotted line represents an optional double bond and X and Y are as hereinabove defined for formula I. Preferably Y represents an alkoxy carbonyl or alkoxysulfonyl group wheren the alkyl moiety has 1 to 12, preferably 1 to 6 carbon atoms. The alkyl groups may be as defined above, i.e. straight, branched or cyclic or they may be straight or branched chains containing a cyclic moiety. In addition, the alkyl moiety of the alkoxycarbonyl groups may be substituted by an amino group,

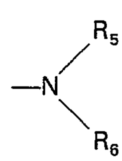
,

as hereinabove defined.

The present invention also provides novel pharmaceutical compositions comprising a compound chosen from the compounds of formula I as defined above and those compounds excluded from formula I by provisos (i) and (ii) in admixture with a pharmaceutically acceptable carrier or excipient.

The compounds of this invention (including these excluded from the definition of formula I by provisos (i) and (ii)) may be prepared by methods generally known in the art.

The preferred process comprises reacting a compound of the general formula III

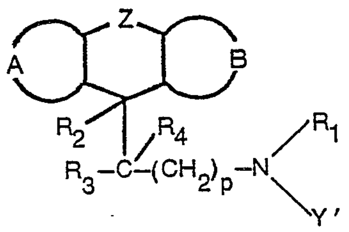

(III)

wherein A, B, Z, $R_1$, $R_2$, $R_3$, $R_4$ and p are as defined for formula I and Y′ represents a replaceable group, with a compound of formula IV

TY (IV),

wherein Y is as defined for formula I and T represents a group capable of being eliminated together with Y′.

Preferably, one of T and Y′ is a reactive in organic or organic ester or ether group, and the other is either hydrogen or methyl.

Thus, for the preparation of compounds of general formula I or, in particular, of formula II wherein Y represents $COOR_7$ with $R_7$ being as defined above, a compound wherein Y′ represents methyl may be reacted with a compound of the formula $TCOOR_7$ wherein T represents halogen, preferably chlorine.

The reaction is conveniently carried out by heating the starting materials in an inert solvent, preferably at reflux temperature. Typical representatives of inert solvents are benzene, toluene, tetrahydrofuran, chloroform etc.

In a further embodiment of the general process outlined above, a compound of formula III wherein Y′ represents H is reacted with a compound of formula $TCOOR_7$ wherein T represents $OR_7$, whereby $R_7$ is as defined above, preferably, however, *t*-butyl.

The compounds of formula I wherein Y represents $SO_2R_8$ are preferably prepared by reacting a compound of formula III wherein Y′ represents H with a compound of formula IV wherein T represents halogen, preferably chlorine, and Y represents $SO_2R_8$. The reaction is preferably carried out at room temperature in an inert solvent such as benzene and toluene in the presence of a base such as $K_2CO_3$, $Na_2CO_3$, NaH, NaOH, triethylamine or other strong organic bases. The inorganic bases are preferred.

As is apparent from the nature of the group Y in the compounds of formula I, certain ester groups obtained according to the procedures outlined above may be transformed into other ester groups by transesterification. Thus for example a compound wherein Y represents —COO 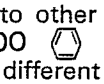 may be reacted with $NaOR_7$ to give a compound wherein Y represents $COOR_7$ ($R_7$ being different from phenyl).

The starting compounds of formula III are partly known compounds which are available on the market, e.g. azatadine:

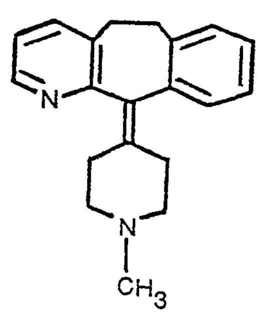

Other starting compounds of formula III may be prepared according to methods described in the literature, e.g. in U.S. Patent 3.326.924 and Belgian Patent 647.043 or according to analogous methods.

As is obvious from the chemical structure, compounds of this invention have assymetric centers (e.g. position 11 in formula II).

In the preparation, a mixture of the optical isomers (d and l), is obtained which can be separated by methods well known in the art.

The following examples illustrate the process variations described above:

Example 1

A. *11-(N-Carboethoxy-4-piperidylidene)-6,11-dihydro-5H-benzo[5,6]cyclohepta[1,2-b]pyridine*

To a solution of 10.9 g (0.1 mole) of ethylchloroformate in 300 ml of anhydrous benzene is added dropwise, with stirring at room temperature, a solution of 14.5 g (0.05M) of 11 - (N - methyl - 4 - piperidylidene) - 6,11 - dihydro - 5H - benzo[5,6]cyclohepta[1,2 - b]pyridine (hereinafter referred to as Compound IIA) in 200 ml of benzene. The solution is stirred and is heated under reflux overnight (16—20 hrs.). The mixture is cooled and is poured into ice water and the organic layer is separated, washed with water, dried, and then concentrated to dryness. The residue is triturated with petroleum ether and a white solid having a melting point of 106—107°C is recrystallized from isopropyl ether after decolorization with decolorizing carbon.

B. *11-(N-Carboethoxy-4-piperidylidene)-8-chloro-6,11-dihydro-5H-benzo[5,6]cyclohepta[1,2-b]-pyridine*

Using the procedure of Example 1A, react 16.2 g of the 8-chloro derivative of Compound IIA and 10.9 g (0.1 mole) of ethylchloroformate to prepare the title compound, having a melting point of 128—130°C. The 7-, 9- and 10-chloro analogues are similarly prepared.

C. *11-(N-Carbomethoxy-4-piperidylidene)-6,11-dihydro-5H-benzo[5,6]cyclohepta[1,2-b]pyridine*

Using the procedure of Example 1A, react 14.5 g of Compound IIA and 9.4 g of methylchloroformate to prepare the title compound, having a melting point of 116—118°C.

D. *11-(β-N-Methyl-N-carboethoxy)ethyl-8-chloro-6,11-dihydro-5H-benzo[5,6]cyclohepta[1,2-b]-pyridine*

Using the procedure of Example 1A react a solution of 15.1 g (0.05 mole) of 11 - (β - dimethylaminoethyl) - 8 - chloro - 6,11 - dihydro - 5H - benzo[5,6]cyclohepta[1,2 - b]pyridine in 300 ml of anhydrous benzene with 10.9 g of ethylchloroformate at room temperature to prepare the title compound.

E. *11-(β-N-Methyl-N-carboethoxy)ethylidene-6,11-dihydro-[5H]-benzo[5,6]cyclohepta[1,2-b]pyridine*

Prepare the title compound by the procedure of Example 1A using 14.9 g of dimethylamino ethylidene - 6,11 - dihydro - [5H] - benzo[5,6]cyclohepta[1,2 - b]pyridine and 10.9 g of ethylchloroformate at room temperature in benzene.

F. *4-(N-Carboethoxy-piperidylidene)4H-benzo[4,5]cyclohepta[1,2-b]thiophene-10(9H)-one*

Using the procedure of Example 1A, react 21.3 g of N - methylpiperidylidene - 4H - benzo[4,5]cyclohepta[1,2 - b]thiophene - 10(9H) - one in 300 ml of benzene with a solution of 10.9 g of ethylchloroformate in 300 ml benzene to prepare the title compound.

Example 2

*11-(N-Carbophenoxy-4-piperidylidene)-6,11-dihydro-5H-benzo[5,6]cyclohepta[1,2-b]pyridine (Compound IIB)*

To a solution of 29.1 g (0.1 mole) of Compound IIA in 150 ml of anhydrous carbon tetrachloride is added 17 g of phenylchloroformate in an equal volume of anhydrous carbon tetrachloride. Heat under reflux for 15 minutes with stirring and pour into water. Separate and wash the organic layer with water and remove solvent. Extract the residue with ether, filter off the insoluble material and remove the ether. The residue is recrystallized from isopropyl ether to yield the title compound having a melting point of 127—130°C. Similarly, prepare the 7-, 8-, 9- or 10-chloro derivatives of the title compound using this procedure.

Example 3

*11-(N-Carboisopropoxy-4-piperidylidene-6,11-dihydro-5H-benzo[5,6]cyclohepta[1,2-b]pyridine*

Dissolve 0.5 g sodium metal in 50 ml isopropanol and add 7.9 g of Compound IIB from Example 2. Heat with stirring for 5 hours on the steam bath at 90—95° and allow to cool overnight.

Add ice water to precipitate the product and extract 3 times with ether and once with chloroform. Wash with water, distill off solvents, triturate with hexane and recrystallize from isopropylether. The melting point is 147—148°C.

Using this procedure, and replacing the isopropanol with n-butanol, cyclopentanol, allyl alcohol, cyclopropylmethanol, benzylalcohol, p-chlorobenzylalcohol, phenethylalcohol, dimethylaminoethylalcohol or N-methyl-4-hydroxy-piperidine prepare the corresponding esters. Similarly, using the 7-, 8-, 9- and 10-chloro derivatives of Compound IIB and the sodium salts of the aforementioned alcohols, prepare the corresponding 7-, 8-, 9- and 10-chloro compounds.

Example 4

*11-(N-Carbo-t-butoxy-4-piperidylidene-6,11-dihydro-5H-benzo[5,6]cyclohepta[1,2-b]pyridine*

Dissolve 13.8 g of 11 - (4 - piperidylidene) - 6,11 - dihydro - 5H - benzo[5,6]cyclohepta-[1,2 - b]pyridine (Compound IIC), prepared according to the method of Villani et al., J. Med. Chem. *15,* 750 (1972), in 250 ml of dry tetrahydrofuran. With stirring, add 12 g of di-butyl carbonate and stir at room temperature overnight. The mixture is poured into water, is extracted with ether and is washed with water and the solvent is removed. Recrystallize the residue from isopropyl ether. The melting point is 144—145°C.

Example 5

*N-Methanesulfonyl-4-piperidylidene-6,11-dihydro-5H-benzo[5,6]cyclohepta[1,2-b]pyridine*

To 10 g of compound IIC in 200 ml of dry toluene add 13 g of anhydrous potassium carbonate. After several minutes of stirring at room temperature, add dropwise a solution of 6 g of methanesulfonyl chloride in 20 ml of toluene. Continue stirring for 16 to 20 hours and then filter. Recrystallize the solid material from ethanol. The melting point is 223—224°C.

Using this procedure and adjusting the weight of the requisite sulfonyl chloride so that 0.04 moles of said alkanesulfonyl chloride are used, the ethanesulfonyl, n-propylsulfonyl, n-butylsulfonyl, cyclopropylsulfonyl, heptylsulfonyl, dodecylsulfonyl, phenylsulfonyl, p-methylphenyl-sulfonyl, p-fluoro-phenylsulfonyl, p-chlorophenylsulfonyl, benzylsulfonyl, p-chlorobenzylsulfonyl, p-tertbutylphenyl-sulfonyl and cyclopentylsulfonyl compounds of formula I wherein Y is $SO_2R$ are obtained. Similarly, prepare the tricyclic ring substituted chloro derivatives.

Substituting the appropriate starting material having a double bond between the 5 and 6 positions of the ring system, and using the procedures set forth in Examples 1 to 5 above (which show the preparation of the 6,11-dihydro compounds), the corresponding 6,11-dehydro compounds are prepared.

Also, by substituting an appropriate bromo or other halo analogue for the 7-, 8-, 9- or 10-chloro-substituted starting material mentioned above, other desired halo compounds of the formula I may be prepared.

Typical representatives of compounds from the preferred group having the general formula II

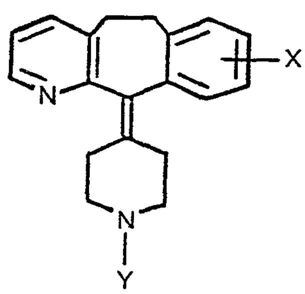

are listed in the table below:

| Compound No. | X | Y | m.p. |
|---|---|---|---|
| 1 | H | $COOC_2H_5$ | 106–107°C |
| 2 | H | $COOCH_2CCl_3$ | 147°C |
| 3 | H | COO 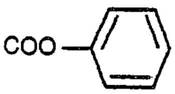 | 127–130°C |
| 4 | H | $COOCH(CH_3)_2$ | 147–148°C |
| 5 | H | $COO(CH_2)_2.N(CH_3)_2$ | 84–87°C |
| 6 | H | $COOCH_3$ | 116–118°C |
| 7 | H | $COO(CH_2)_3CH_3$ | 86–89°C |
| 8 | H | $COOC(CH_3)_3$ | 144–145°C |
| 9 | H | COO 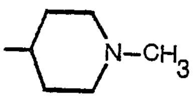 | 158–160°C |
| 10 | H | $COOCH_2$ 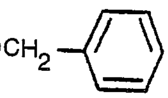 | 106–108°C |
| 11 | 8–Cl | $COOC_2H_5$ | 128–130°C |
| 12 | H | $COOCH_2CH{=}CH_2$ | 88–91°C |
| 13 | H | $COOCH_2$ 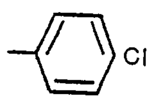 | 131–134°C |
| 14 | H | $COO(CH_2)_2$ 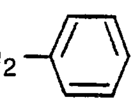 | 101–104°C |
| 15 | H | $COOCH_2$ 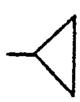 | 126–127°C |
| 16 | H | COO 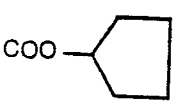 | 162–164°C |
| 17 | H | $SO_2$ 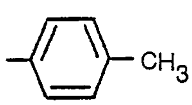 | 197–199°C |

| Compound No. | X | Y | m.p. |
|---|---|---|---|
| 18 | 8–Cl | 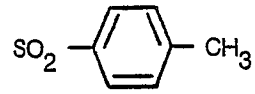 | 185–187°C |
| 19 | H | 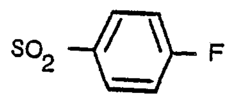 | 186–187°C |
| 20 | H | $SO_2CH_3$ | 223–224°C |
| 21 | H | $SO_2(CH_2)_2CH_3$ | 183–184°C |
| 22 | H | $SO_2CH_2CH_3$ | 173–174°C |
| 23 | H | $SO_2(CH_2)_3CH_3$ | 185–186°C |
| 24 | H | 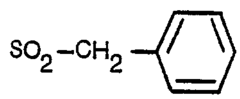 | 185–186°C |
| 25 | H | 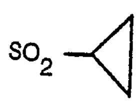 | 157–158°C |

The compounds of the present invention (including those excluded from the definition of formula I by provisos (i) and (ii)) are useful as non-sedating antihistamines. Such compounds act as anti-allergic agents in the treatment of such conditions as perennia; and seasonal allergic rhinitis and chronic urticaria, and can be administered in pharmaceutical formulations comprising the compound in admixture with a pharmaceutical carrier suitable for enteral or parenteral administration. The formulations may be in solid form, as for example tablets and capsules, or in liquid form as for example syrups, elixirs, emulsions, and injectables. In the formulation of pharmaceutical dosage forms there generally is utilized excipients, for example, water, gelatin, lactose, starches, magnesium stearate, talc, vegetable oils, benzyl alcohols, gums, polyalkylene glycols, and petroleum jelly. Preferred formulations are more fully illustrated in Example 6.

Although the required dosage will be determined by such factors as the patient's age, sex, weight and the severity of the allergic reaction to be treated, the preferred human dosage range is likely to be 4 to 50 mg of the effective compound 1 to 3 times per day. The preferred dosage ranges for other animals can readily be determined by using standard testing methods.

The following data show the pharmaceutical effect of the compounds of this invention. (The data were elåborated with the compound 8 - chloro - 6,11 - dihydro - 11 - (1 - carboethoxy - 4 - piperidylidene) - 5 - *H* - benzo[5,6]cyclohepta[1,2 - b]pyridine).

Oral $ED_{50}$ for preventing histamine induced lethality in guinea pigs is 0.19 mg/kg. The acute toxicity is very low (e.g. >320 mg/kg in mice; >60 mg/kg in dogs etc.). Oral $ED_{50}$ for preventing histamine-induced paw edema in mice is 1.3 mg/kg.

The compound showed very little or no CNS activity in mice, rats, dogs or monkeys, eg. no physostigmine lethality in mice at doses up to 320 mg/kg; no overt behavioral or neurologic or autonomic effects in mice or rats after doses of 10—300 mg/kg, in dogs after doses of 15—60 mg/kg or in monkeys at doses of 30—90 mg/kg.

The following examples are illustrative of the aforementioned pharmaceutical compositions:

Example 6

A syrup comprising a compound of the present invention (Active Compound), e.g. 11 - (N - carboethoxy - 4 - piperidylidene) - 8 - chloro - 6,11 - dihydro - 5H - benzo[5,6]cyclohepta[1,2 -

b]pyridine, 11 - (N - methanesulfonyl - 4 - piperidylidene) - 6,11 - dihydro - 5H - benzo[5,6]cyclohepta[1,2 - b]pyridine, 11 - (N - carboethoxy - 4 - piperidylidene) - 6,11 - dihydro - 5H - benzo[5,6]cyclohepta[1,2 - b]pyridine, 11 - (N - carbomethoxy - 4 - piperidylidene) - 6,11 - dihydro - 5H - benzo[5,6]cyclohepta[1,2 - b]pyridine or 11 - (N - carbophenoxy - 4 - piperidylidene) - 6,11 - dihydro - 5H - benzo[5,6]cyclohepta[1,2 - b]pyridine, is prepared from the following ingredients:

| | per ml |
|---|---|
| Active Compound | 0.100 mg |
| Sucrose | 600 mg |
| Sorbitol | 140 mg |
| Propylene Glycol | 20.0 mg |
| Methylparaben | 1.00 mg |
| Propylparaben | 0.200 mg |
| F.D. & C. Yellow No. 6 | 0.225 mg |
| Alcohol USP | 0.0021 ml |
| Imitation Black Currant Flavor | 0.001 ml |
| Purified Water USP | q.s. |
| | 1.0 ml |

The syrup is prepared by combining the above ingredients according to standard techniques.

Example 7

A tablet comprising a compound of the present invention (Active Compound) is prepared by a spray-dry process from the following ingredients:

| Component I | Tablet I (mg/tablet) | Tablet II (mg/tablet) |
|---|---|---|
| Active Compound* | 1.00 | 10.00 |
| Lactose, Hydrous USP (Impalpable Powder) | 212 | 180.00 |
| Povidone NF | 10.0 | — |
| Corn Starch (Food Grade) | 15.0 | 6.0 |
| Purified Water USP (Evaporates) | 0.102 ml | 0.1 |
| Additional Components | | |
| Corn Starch (Food Grade) | 11.5 | 6.0 |
| Magnesium Stearate USP | 0.500 | 1.2 |
| Gelatine | — | 2.5 |

The materials of Component I are combined and spray dried by standard techniques. The resulting spray dried material is combined with the additional components listed above and processed to form tablets.

*) e.g. 11 - (N - carboethoxy - 4 - piperidylidene) - 8 - chloro - 6,11 - dihydro - 5H - benzo[5,6]cyclohepta[1,2 - b]pyridine.

**Claims for the Contracting States: BE CH DE FR GB IT LI LU NL SE**

1. Compounds of the general formula I

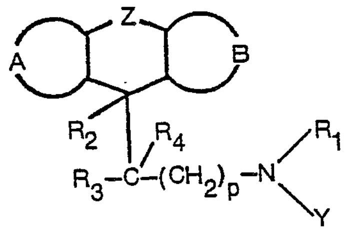

(I)

wherein

A represents the group of atoms required for completing a fused aromatic 5- or 6-membered heterocyclic ring or a fused benzene ring;

B represents the group of atom required for completing a fused pyridine, benzene or X-substituted benzene ring wherein X is halogen, with the proviso that, when A defines a nitrogen-containing heterocyclic ring, then B defines a fused benzene or X-substituted benzene ring;

Z represents a single bond or a substituted or unsubstituted methylene or ethylene group, the substituent being an oxo or hydroxy group, or Z represents vinylene;

$R_2$ represents hydrogen or an alkyl group having 1 to 3 carbon atoms and

$R_3$ represents hydrogen, or

$R_2$ and $R_3$ form together a second bond;

$R_4$ represents hydrogen and

$R_1$ represents an alkyl group having 1 to 6 carbon atoms, or

$R_1$ and $R_4$ form together a second bridge $—(CH_2)_2—$ between the carbon atom and the nitrogen atom to which they are attached;

p represents 1 or 2; and

Y represents either of the groups $—COOR_7$ and $—SO_2R_8$

wherein

$R_7$ represents alkyl, cycloalkyl, cycloalkylalkyl, alkenyl, phenyl, phenylloweralkyl or 2-, 3- or 4-piperidyl and

$R_8$ represents alkyl, cycloalkyl, cycloalkylalkyl, alkenyl, phenyl or phenylloweralkyl, whereby the alkyl, cycloalkyl, cycloalkylalkyl and alkenyl groups represented by $R_7$ may optionally be substituted by the group

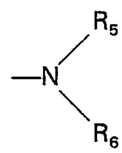

wherein $R_5$ and $R_6$ each represent hydrogen or an alkyl group having 1 to 6 carbon atoms, the piperidyl groups represented by $R_7$ may be substituted at the nitrogen atom by a lower alkyl group having 1 to 4 carbon atoms, and whereby the phenyl groups and the phenyl moiety of the phenylloweralkyl groups represented by $R_7$ and/or $R_8$ may be substituted by halogen or lower alkyl having 1 to 4 carbon atoms, subject to the following further provisos:

(i) that when A is a group of atoms required to form a fused [1,2-b] thiophene ring, Z is $—CH_2—CH_2—$ or $—CH{=}CH—$, p is 2 and either

1) B is a group of atoms required to form a fused benzene ring, $R_2$ and $R_3$ are both hydrogen or form a double bond, $R_4$ is hydrogen and $R_1$ is $C_1—C_4$ alkyl or $R_4$ and $R_1$ form the second bridge $(—CH_2—)_2$; or

2) B is an X substituted benzene ring, $R_2$ and $R_3$ form a double bond, $R_4$ is hydrogen and $R_1$ is Me; then $R_7$ cannot be $C_1—C_4$ alkyl or phenyl ($C_1—C_4$ alkyl); and

(ii) when A and B represent respectively a fused benzene ring and a fused benzene or X substituted benzene ring then $R_1$ and $R_4$ form together a second bridge $\text{-}(CH_2\text{-})_2$ between the carbon atom and nitrogen atom to which they are attached:

and wherein, in Y, the alkyl and alkenyl groups have up to 12 carbon atoms; the loweralkyl groups (in the phenylloweralkyl groups) have 1 to 4 carbon atoms, the cycloalkyl groups have 3 to 7 carbon atoms, and the cycloalkylalkyl groups have 4 to 12 carbon atoms.

2. Compounds according to claim 1, wherein Z represents an ethylene or vinylene group.

3. Compounds according to claim 1 or 2, wherein $R_2$ and $R_3$ together form a second bond.

4. Compounds according to any one of claims 1 to 3, wherein $R_1$ and $R_4$ together form a bridge of the formula $—(CH_2)_2—$.

5. Compounds according to any one of claims 1 to 4, wherein the ring defined by A is a thiophene or pyridine ring.

6. Compounds according to any one of claims 1 to 5, wherein the ring defined by B is a fused benzene or X-substituted benzene ring with X being as defined in claim 1.

7. Compounds according to any one of claims 1 to 6 having the general formula II

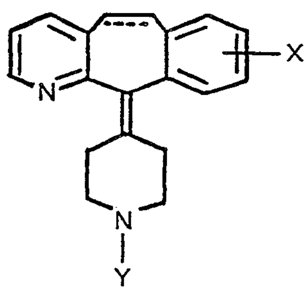

(II)

wherein the dotted line represents an optional double bond and X and Y are as defined in claim 1.

8. Compounds according to any one of claims 1 to 7, wherein there is a single bond between the 5 and 6-positions.

9. Compounds according to any one of claims 1 to 8, wherein X represents hydrogen, 8-chlorine or 8-bromine.

10. Compounds according to any one of claims 1 to 9, said compounds being
11-(N-carboethoxy-4-piperidylidene)-8-chloro-6,11-dihydro-5H-benzo[5,6]cyclohepta[1,2-b]pyridine;
11-(N-methanesulfonyl-4-piperidylidene)-6,11-dihydro-5H-benzo[5,6]cyclohepta[1,2-b]pyridine;
11-(N-carboethoxy-4-piperidylidene)-6,11-dihydro-5H-benzo[5,6]cyclohepta[1,2-b]pyridine;
11-(N-carbomethoxy-4-piperidylidene)-6,11-dihydro-5H-benzo[5,6]cyclohepta[1,2-b]pyridine; or
11-(N-carbophenoxy-4-piperidylidene)-6,11-dihydro-5H-benzo[5,6]cyclohepta[1,2-b]pyridine.

11. Pharmaceutical compositions comprising as an active ingredient a compound chosen from compounds of formula I as defined in claim 1 and those compounds excluded from claim 1 by provisos (i) and (ii) therein, in admixture with a pharmaceutically acceptable carrier or excipient.

12. Process for preparing compounds of formula I as defined in claim 1, characterized in that a compound of the general formula III

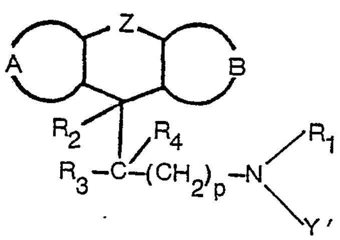

III

wherein A, B, Z, $R_1$, $R_2$, $R_3$, $R_4$ and p are as defined in claim 1 and Y′ represents a replaceable group, with a compound of formula IV

$$TY \qquad (IV),$$

wherein Y is as defined in claim 1 and T represents a group capable of being eliminated together with Y′, and that, if desired, a so obtained compound of formula I is transferred into another compound of formula I by transesterification of the group Y.

13. The compounds of formula I as defined in claim 1 and those excluded from claim 1 by provisos (i) and (ii) therein for use as antihistamines.

**Claims for the Contracting State: AT**

1. Process for the preparation of compounds of the general formula I

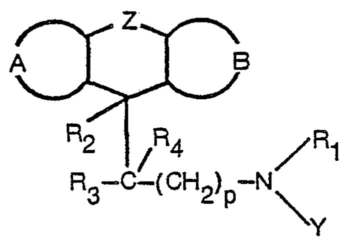

(I)

wherein

A represents the group of atoms required for completing a fused aromatic 5- or 6-membered heterocyclic ring or a fused benzene ring;

B represents the group of atoms required for completing a fused pyridine, benzene or X-substituted benzene ring wherein X is halogen, with the proviso that, when A defines a nitrogen-containing heterocyclic ring, then B defines a fused benzene or X-substituted benzene ring;

Z represents a single bond or a substituted or unsubstituted methylene or ethylene group, the substituent being an oxo or hydroxy group, or Z represents vinylene;

$R_2$ represents hydrogen or an alkyl group having 1 to 3 carbon atoms and

$R_3$ represents hydrogen, or

$R_2$ and $R_3$ form together a second bond;

$R_4$ represents hydrogen and

$R_1$ represents an alkyl group having 1 to 6 carbon atoms, or

$R_1$ and $R_4$ form together a second bridge $—(CH_2)_2—$ between the carbon atom and the nitrogen atom to which they are attached;

p represents 1 or 2; and

Y represents either of the groups $—COOR_7$ and $—SO_2R_8$ wherein

$R_7$ represents alkyl, cycloalkyl, cycloalkylalkyl, alkenyl, phenyl, phenylloweralkyl or 2-, 3- or 4-piperidyl and

$R_8$ represents alkyl, cycloalkyl, cycloalkylalkyl, alkenyl, phenyl or phenylloweralkyl, whereby the alkyl, cycloalkyl, cycloalkylalkyl and alkenyl groups represented by $R_7$ may optionally be substituted by the group

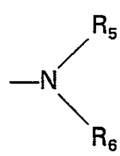

wherein $R_5$ and $R_6$ each represent hydrogen or an alkyl group having 1 to 6 carbon atoms, the piperidyl groups represented by $R_7$ may be substituted at the nitrogen atom by a lower alkyl group having 1 to 4 carbon atoms, and whereby the phenyl groups and the phenyl moiety of the phenylloweralkyl groups represented by $R_7$ and/or $R_8$ may be substituted by halogen or lower alkyl having 1 to 4 carbon atoms, subject to the following further provisos:

(i) that when A is a group of atoms required to form a fused [1,2-b] thiophene ring, Z is $—CH_2—CH_2—$ or $—CH{=}CH—$, p is 2 and either

1) B is a group of atoms required to form a fused benzene ring, $R_2$ and $R_3$ are both hydrogen or form a double bond, $R_4$ is hydrogen and $R_1$ is $C_1—C_4$ alkyl or $R_4$ and $R_1$ form the second bridge $(—CH_2—)_2$; or

2) B is an X substituted benzene ring, $R_2$ and $R_3$ form a double bond, $R_4$ is hydrogen and $R_1$ is Me; then $R_7$ cannot be $C_1—C_4$ alkyl or phenyl ($C_1—C_4$ alkyl); and

(ii) when A and B represent respectively a fused benzene ring and a fused benzene or X substituted benzene ring then $R_1$ and $R_4$ form together a second bridge $+CH_2+_2$ between the carbon atom and nitrogen atom to which they are attached;

and wherein, in Y, the alkyl and alkenyl groups have up to 12 carbon atoms, the loweralkyl groups (in the phenylloweralkyl groups) have 1 to 4 carbon atoms, the cycloalkyl groups have 3 to 7 carbon atoms, and the cycloalkylalkyl groups have 4 to 12 carbon atoms, characterised in that a compound of the general formula III

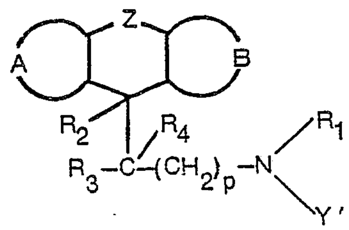

(III)

wherein A, B, Z, $R_1$, $R_2$, $R_3$, $R_4$ and p are as defined in claim 1 and Y′ represents a replaceable group, with a compound of formula IV

$$TY \qquad (IV),$$

wherein Y is as defined in claim 1 and T represents a group capable of being eliminated together with Y′, and that, if desired, a so obtained compound of formula I is transferred into another compound of formula I by transesterification of the group Y.

2. Process according to claim 1, characterized in that a compound of formula III wherein Y′ is methyl is reacted with a compound of the formula

$$T\ COOR_7$$

with T being halogen, preferably chlorine, and $R_7$ being as defined in claim 1.

3. Process according to claim 1, characterized in that a compound of formula III wherein Y′ is hydrogen is reacted with a compound of the formula

$$T\ COOR_7$$

with T representing the group $OR_7$ and $R_7$ being as defined in claim 1.

4. Process according to claim 1, characterized in that a compound of formula III wherein Y′ represents hydrogen is reacted with a compound of formula IV wherein T represents halogen, preferably chlorine, and Y represents the group $SO_2R_8$ with $R_8$ being as defined in claim 1.

5. Process according to any one of claims 1 to 4, characterized in that as a starting compound is used a compound of the formula II′

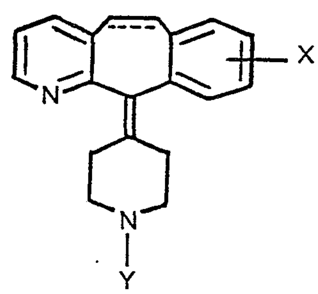

(II′)

wherein X is as defined in claim 1 and Y′ is as defined in any one of claims 1 to 4.

6. Process according to claim 5, characterized in that a compound of formula II′ wherein X represents hydrogen or 8-chlorine is reacted with a compound of the formula

$$TCOOC_2H_5$$

whereby Y′ and T are as defined in any one of claims 1 to 4.

7. A process for making a pharmaceutical composition which comprises mixing a compound chosen from the compounds of formula I as defined in claim 1 and those compounds excluded from claim 1 by provisos (I) and (II) therein, in admixture with a pharmaceutically acceptable carrier or excipient.

**Patentansprüche für die Vertragsstaaten: BE CH DE FR GB IT LI LU NL SE**

1. Verbindungen der allgemeinen Formel I

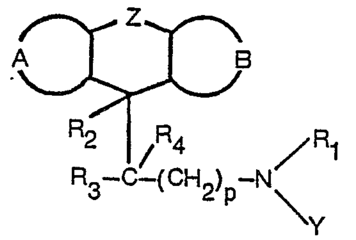

(I)

in der

A die zur Vervollständigung eines anellierten aromatischen 5- oder 6-gliedrigen heterocyclischen Rings oder eines anellierten Benzol-Rings erforderliche Gruppe von Atomen bezeichnet,

B die zur Vervollständigung eines anellierten Pyridin-, Benzol- oder X-substituierten Benzol-Rings, worin X Halogen ist, erforderliche Gruppe von Atomen bezeichnet, mit der Maßgabe, daß B einen anellierten Benzol- oder X-substituierten Benzol-Ring darstellt, wenn A für einen stickstoffhaltigen heterocyclischen Ring steht,

Z eine Einfach-Bindung oder eine substituierte oder unsubstituierte Methylen- oder Ethylen-Gruppe bezeichnet, worin der Substituent eine Oxo- oder Hydroxy-Gruppe ist, oder Z Vinylen bezeichnet,

$R_2$ Wasserstoff oder eine Alkyl-Gruppe mit 1 bis 3 Kohlenstoff-Atomen bezeichnet und

$R_3$ Wasserstoff bezeichnet oder

$R_2$ und $R_3$ gemeinsam eine zweite Bindung bilden,

$R_4$ Wasserstoff bezeichnet und

$R_1$ eine Alkyl-Gruppe mit 1 bis 6 Kohlenstoff-Atomen bezeichnet oder

$R_1$ und $R_4$ gemeinsam eine zweite Brücke $-(CH_2)_2-$ zwischen dem Kohlenstoff-Atom und dem Stickstoff-Atom, an die sie jeweils gebunden sind, bilden,

p für 1 oder 2 steht,

Y eine der Gruppen $-COOR_7$ und $-SO_2R_8$ bezeichnet, worin

$R_7$ für Alkyl, Cycloalkyl, Cycloalkylalkyl, Alkenyl, Phenyl, Phenylniederalkyl oder 2-, 3- oder 4-Piperidyl steht und

$R_8$ für Alkyl, Cycloalkyl, Cycloalkylalkyl, Alkenyl, Phenyl oder Phenylniederalkyl steht,

wobei die durch $R_7$ dargestellten Alkyl-, Cycloalkyl-, Cycloalkylalkyl- oder Alkenyl-Gruppen gegebenenfalls substituiert sein können durch die Gruppe

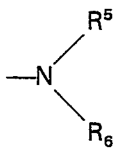 ,

in der $R_5$ und $R_6$ jeweils für Wasserstoff oder eine Alkyl-Gruppe mit 1 bis 6 Kohlenstoff-Atomen stehen, wobei die durch $R_7$ dargestellte Piperidyl-Gruppe an dem Stickstoff-Atom durch eine niedere Alkyl-Gruppe mit 1 bis 4 Kohlenstoff-Atomen substituiert sein kann und wobei die durch $R_7$ und/oder $R_8$ dargestellten Phenyl-Gruppen und die Phenyl-Struktureinheiten der Phenylniederalkyl-Gruppen durch Halogen oder Niederalkyl mit 1 bis 4 Kohlenstoff-Atomen substituiert sein können,

mit den weiteren Maßgaben, daß,

(i) wenn A eine zur Bildung eines anellierten [1,2-b]-Thiophen-Rings erforderliche Gruppe ist, Z $-CH_2-CH_2-$ oder $-CH=CH-$ ist, p 2 ist und entweder

1) B eine zur Bildung eines anellierten Benzol-Rings erforderliche Gruppe von Atomen ist, $R_2$ und $R_3$ beide Wasserstoff sind oder eine Doppelbindung bilden, $R_4$ Wasserstoff ist und $R_1$ $C_1-C_4$-Alkyl ist oder $R_4$ und $R_1$ die zweite Brücke $(-CH_2-)_2$ bilden oder

2) B eine X-substituierter Benzol-Ring ist, $R_2$ und $R_3$ eine Doppelbindung bilden, $R_4$ Wasserstoff ist und $R_1$ Me ist,

dann $R_7$ nicht $C_1-C_4$-Alkyl oder Phenyl ($C_1-C_4$-alkyl) sein kann, und

(ii) wenn A und B jeweils einen anellierten Benzol-Ring und einen anellierten Benzol- oder X-substituierten Benzol-Ring bezeichnen,

dann $R_1$ und $R_4$ zusammen eine zweite Brücke $(-CH_2-)_2$ zwischen dem Kohlenstoff-Atom und dem Stickstoff-Atom, an die sie jeweils gebunden sind, bilden,

und worin in Y die Alkyl- und Alkenyl-Gruppen bis zu 12 Kohlenstoff-Atome, die Niederalkyl-Gruppen

(in den Phenylniederalkyl-Gruppen) 1 bis 4 Kohlenstoff-Atome, die Cycloalkyl-Gruppen 3 bis 7 Kohlenstoff-Atome und Cycloalkylalkyl-Gruppen 4 bis 12 Kohlenstoff-Atome aufweisen.

2. Verbindung nach Anspruch 1, dadurch gekennzeichnet, daß eine Ethylen- oder Vinylen-Gruppe bezeichnet.

3. Verbindungen nach Anspruch 1 oder 2, dadurch gekennzeichnet, daß $R_2$ und $R_3$ zusammen eine zweite Bindung bilden.

4. Verbindungen nach irgendeinem der Ansprüche 1 bis 3, dadurch gekennzeichnet, daß $R_1$ und $R_4$ zusammen eine Brücke der Formel $-(CH_2)_2-$ bilden.

5. Verbindungen nach irgendeinem der Ansprüche 1 bis 4, dadurch gekennzeichnet, daß der durch A bezeichnete Ring eine Thiophen- oder Pyridin-Ring ist.

6. Verbindungen nach irgendeinem der Ansprüche 1 bis 5, dadurch gekennzeichnet, daß der durch B bezeichnete Ring ein anellierter Benzol- oder X-substituierter Benzol-Ring ist, wobei X die in Anspruch 1 angegebene Bedeutung hat.

7. Verbindungen nach irgendeinem der Ansprüche 1 bis 6 mit der allgemeinen Formel II

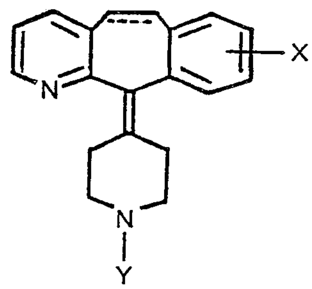

(II)

in der
die gestrichelte Linie eine wahlweise Doppelbindung bezeichnet und X und Y die in Anspruch 1 angegebenen Bedeutungen haben.

8. Verbindungen nach irgendeinem der Ansprüche 1 bis 7, dadurch gekennzeichnet, daß sich zwischen der 5- und der 6-Stellung eine Einfach-Bindung befindet.

9. Verbindungen nach irgendeinem der Ansprüche 1 bis 8, dadurch gekennzeichnet, daß X für Wasserstoff, 8-Chlor oder 8-Brom steht.

10. Verbindungen nach irgendeinem der Ansprüche 1 bis 9, nämlich
11-(N-Carboethoxy-4-piperidyliden)-8-chloro-6,11-dihydro-5H-benzo[5,6]cyclohepta[1,2-b]pyridin,
11-(N-Methansulfonyl-4-piperidyliden)-6,11-dihydro-5H-benzo[5,6]cyclohepta [1,2-b]pyridin,
11-(N-Carboethoxy-4-piperidyliden)-6,11-dihydro-5H-benzo[5,6]cyclohepta[1,2-b]pyridin,
11-(N-Carbomethoxy-4-piperidyliden)-6,11-dihydro-5H-benzo[5,6]cyclohepta [1,2-b]pyridin oder
11-(N-Carbophenoxy-4-piperidyliden)-6,11-dihydro-5H-benzo[5,6]cyclohepta[1,2-b]pyridin.

11. Pharmazeutische Zusammensetzungen, enthaltend als Wirkstoff eine Verbindung ausgewählt aus Verbindungen der Formel I wie in Anspruch 1 definiert und solchen Verbindungen, die durch die in Anspruch 1 genannten Maßgaben (i) und (ii) ausgeschlossen sind, im Gemisch mit einem pharmazeutisch unbedenklichen Träger oder Streckmittel.

12. Verfahren zur Herstellung von Verbindungen der Formel I nach Anspruch 1, dadurch gekennzeichnet, daß eine Verbindung der Formel III

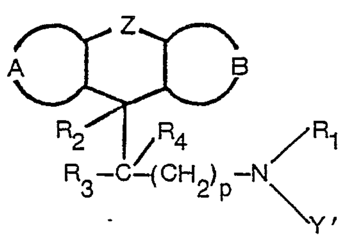

(III)

in der A, B, Z, $R_1$, $R_2$, $R_3$, $R_4$ und p die in Anspruch 1 angegebenen Bedeutungen haben und Y' eine ersetzbare Gruppe bezeichnet, mit einer Verbindung der Formel IV

$$TY \qquad (IV),$$

in der Y die in Anspruch 1 angegebene Bedeutung hat und T eine Gruppe bezeichnet, die sich zusammen mit Y' eliminieren läßt, umgesetzt wird und daß gewünschtenfalls eine so erhaltene Verbindung der Formel I durch Umesterung der Gruppe Y in eine andere Verbindung der Formel I umgewandelt wird.

13. Verwendung der Verbindungen der Formel I nach Anspruch 1 und solcher Verbindungen, die durch die in Anspruch 1 genannten Maßgaben (i) und (ii) ausgeschlossen sind, als Antihistamine.

**Patentansprüche für den Vertragsstaat: AT**

1. Verfahren zur Herstellung von Verbindungen der allgemeinen Formel I

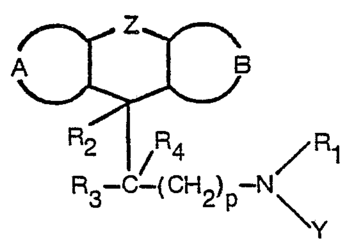

(I)

in der

A die zur Vervollständigung eines anellierten aromatischen 5- oder 6-gliedrigen heterocyclischen Rings oder eines anellierten Benzol-Rings erforderliche Gruppe von Atomen bezeichnet,

B die zur Vervollständigung eines anellierten Pyridin-, Benzol- oder X-substituierten Benzol-Rings, worin X Halogen ist, erforderliche Gruppe von Atomen bezeichnet, mit der Maßgabe, daß B einen anellierten Benzol- oder X-substituierten Benzol-Ring darstellt, wenn A für einen stickstoffhaltigen heterocyclischen Ring steht,

Z eine Einfach-Bindung oder eine substituierte oder unsubstituierte Methylen- oder Ethylen-Gruppe bezeichnet, worin der Substituent eine Oxo- oder Hydroxy-Gruppe ist, oder Z Vinylen bezeichnet,

$R_2$ Wasserstoff oder eine Alkyl-Gruppe mit 1 bis 3 Kohlenstoff-Atomen bezeichnet und

$R_3$ Wasserstoff bezeichnet oder

$R_2$ und $R_3$ gemeinsam eine zweite Bindung bilden,

$R_4$ Wasserstoff bezeichnet und

$R_1$ eine Alkyl-Gruppe mit 1 bis 6 Kohlenstoff-Atomen bezeichnet oder

$R_1$ und $R_4$ gemeinsam zweite Brücke $-(CH_2)_2-$ zwischen dem Kohlenstoff-Atom und dem Stickstoff-Atom, an die sie jeweils gebunden sind, bilden,

p für 1 oder 2 steht,

Y eine der Gruppen $-COOR_7$ und $-SO_2R_8$ bezeichnet, worin

$R_7$ für Alkyl, Cycloalkyl, Cycloalkylalkyl, Alkenyl, Phenyl, Phenylniederalkyl oder 2-, 3- oder 4-Piperidyl steht und

$R_8$ für Alkyl, Cycloalkyl, Cycloalkylalkyl, Alkenyl, Phenyl oder Phenylniederalkyl steht,

wobei die durch $R_7$ dargestellten Alkyl-, Cycloalkyl-, Cycloalkylalkyl- oder Alkenyl-Gruppen gegebenenfalls substituiert sein können durch die Gruppe

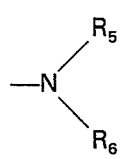

,

in der $R_5$ und $R_6$ jeweils für Wasserstoff oder eine Alkyl-Gruppe mit 1 bis 6 Kohlenstoff-Atomen stehen, wobei die durch $R_7$ dargestellte Piperidyl-Gruppe an dem Stickstoff-Atom durch eine niedere Alkyl-Gruppe mit 1 bis 4 Kohlenstoff-Atomen substituiert sein kann und wobei die durch $R_7$ und/oder $R_8$ dargestellten Phenyl-Gruppen und die Phenyl-Struktureinheiten der Phenylniederalkyl-Gruppen durch Halogen oder Niederalkyl mit 1 bis 4 Kohlenstoff-Atomen substituiert sein können,

mit den weiteren Maßgaben, daß,

(i) wenn A eine zur Bildung eines anellierten [1,2-b]-Thiophen-Rings erforderliche Gruppe ist, Z $-CH_2-CH_2-$ oder $-CH{=}CH-$ ist, p 2 ist und entweder

1) B eine zur Bildung eines anellierten Benzol-Rings erforderliche Gruppe von Atomen ist, $R_2$ und $R_3$ beide Wasserstoff sind oder eine Doppelbindung bilden, $R_4$ Wasserstoff ist und $R_1$ $C_1-C_4$-Alkyl ist oder $R_4$ und $R_1$ die zweite Brücke $(-CH_2-)_2$ bilden oder

2) B ein X-substituierter Benzol-Ring ist, $R_2$ und $R_3$ eine Doppelbindung bilden, $R_4$ Wasserstoff ist und $R_1$ Me ist,

dann $R_7$ nich $C_1-C_4$-Alkyl oder Phenyl $(C_1-C_4$-alkyl) sein kann, und

(ii) wenn A und B jeweils einen anellierten Benzol-Ring und einen anellierten Benzol- oder X-substituierten Benzol-Ring bezeichnen,

dann $R_1$ und $R_4$ zusammen eine zweite Brücke $(-CH_2-)_2$ zwischen dem Kohlenstoff-Atom und dem Stickstoff-Atom, an die sie jeweils gebunden sind, bilden,

und worin in Y die Alkyl- und Alkenyl-Gruppen bis zu 12 Kohlenstoff-Atome, die Niederalkyl-Gruppen (in den Phenylniederalkyl-Gruppen) 1 bis 4 Kohlenstoff-Atome, die Cycloalkyl-Gruppen 3 bis 7 Kohlenstoff-Atome und Cycloalkylalkyl-Gruppen 4 bis 12 Kohlenstoff-Atome aufweisen, dadurch gekennzeichnet, daß eine Verbindung der Formel III

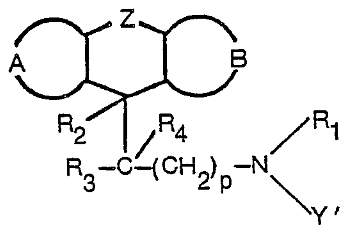

(III)

in der A, B, Z, $R_1$, $R_2$, $R_3$, $R_4$ und p die oben angegebenen Bedeutungen haben und Y' eine ersetzbare Gruppe bezeichnet, mit einer Verbindung der Formel IV

TY (IV),

in der Y die oben angegebene Bedeutung hat und T eine Gruppe bezeichnet, die sich zusammen mit Y' eliminieren läßt, umgesetzt wird und daß gewünschtenfalls eine so erhaltene Verbindung der Formel I durch Umesterung der Gruppe Y in eine andere Verbindung der Formel I umgewandelt wird.

2. Verfahren nach Anspruch 1, dadurch gekennzeichnet, daß eine Verbindung der Formel III, in der Y' Methyl ist, mit einer Verbindung der Formel

$TCOOR_7$

umgesetzt wird, in der T Halogen, vorzugsweise Chlor, ist und $R_7$ die in Anspruch 1 angegebene Bedeutung hat.

3. Verfahren nach Anspruch 1, dadurch gekennzeichnet, daß eine Verbindung der Formel III, in der Y' Wasserstoff ist, mit einer Verbindung der Formel

$TCOOR_7$

umgesetzt wird, in der T für die Gruppe $OR_7$ steht und $R_7$ die in Anspruch 1 angegebene Bedeutung hat.

4. Verfahren nach Anspruch 1, dadurch gekennzeichnet, daß eine Verbindung der Formel III, in der Y' Wasserstoff ist, mit einer Verbindung der Formel IV umgesetzt wird, in der T Halogen, vorzugsweise Chlor, bezeichnet und Y für die Gruppe $SO_2R_8$ steht, in der $R_8$ die in Anspruch 1 angegebene Bedeutung hat.

5. Verfahren nach irgendeinem der Ansprüche 1 bis 4, dadurch gekennzeichnet, daß als Ausgangs-Verbindung eine Verbindung der Formel II'

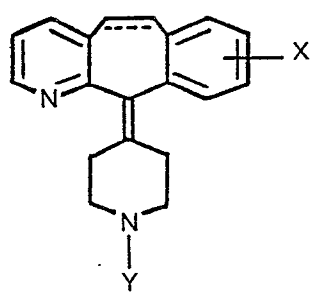

(II')

eingesetzt wird, in der X die in Anspruch 1 angegebene Bedeutung hat und Y' die in irgendeinem der Ansprüche 1 bis 4 angegebene Bedeutung hat.

6. Verfahren nach Anspruch 5, dadurch gekennzeichnet, daß eine Verbindung der Formel II', in der X für Wasserstoff oder 8-Chlor steht, mit einer Verbindung der Formel

$TCOOC_2H_5$

umgesetzt wird, in der Y′ und T die in irgendeinem der Ansprüche 1 bis 4 angegebenen Bedeutungen haben.

7. Verfahren zur Herstellung einer pharmazeutischen Zusammensetzung, dadurch gekennzeichnet, daß eine Verbindung, die ausgewählt ist aus den Verbindungen der Formel I nach Anspruch 1 und solchen Verbindungen, die in Anspruch 1 durch die dort genannten Maßgaben (i) und (ii) ausgeschlossen sind, mit einem pharmazeutisch unbedenklichen Träger oder Streckmittel vemischt wird.

**Revendications pour les Etats contractants: BE CH DE FR GB IT LI LU NL SE**

1. Composés caractérisés en ce qu'ils sont représentés par la formule générale I

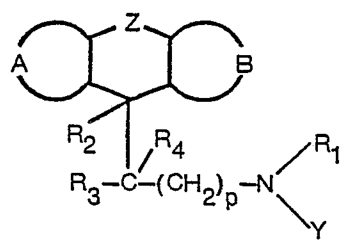

(I)

dans laquelle

A représente le groupe d'atomes requis pour former un anneau condensé aromatique hétérocyclique à 5 ou 6 membres ou un anneau benzène condensé;

B représente le groupe d'atomes requis pour former un anneau condensé pyridine, benzène ou benzène X-substitué dans lequel X est un halogène, avec la condition que B représente un anneau condensé benzène ou benzène X-substitué quand A représente un anneau hétérocyclique contenant de l'azote;

Z représente une liaison simple ou un groupe méthylène ou éthylène substitué ou non-substitué, le substituant étant un groupe oxo ou hydroxy, ou bien Z représente un vinylène;

$R_2$ représente l'hydrogène ou un groupe alkyle ayant 1 à 3 atomes de carbone;

$R_3$ représente l'hydrogène, ou

$R_2$ et $R_3$ forment ensemble une seconde liaison;

$R_4$ représente l'hydrogène;

$R_1$ représente un groupe alkyle ayant 1 à 6 atomes de carbone, ou

$R_1$ et $R_4$ forment ensemble un second pont $—(CH_2)_2—$ entre l'atome de carbone et l'atome d'azote auxquels ils sont fixés;

p représente 1 ou 2;

Y représente l'un des groupes $—COOR_7$ ou $—SO_2R_8$ dans lesquels

$R_7$ représente un groupe alkyle, cycloalkyle, cycloalkylalkyle, alkényle, phényle, phénylalkyle inférieur ou 2-, 3- ou 4- pipéridyle et

$R_8$ représente un groupe alkyle, cycloalkyle, cycloalkylalkyle, alkényle, phényle ou phénylalkyle inférieur, dans lequel les groupes alkyles, cycloalkyles, cycloalkylalkyles et alkényles représentés par $R_7$ peuvent être éventuellement substitués par le groupe

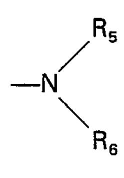

dans lequel $R_5$ et $R_6$ représentent chacun l'hydrogène ou un groupe alkyle ayant 1 à 6 atomes de carbone, les groupes pipéridyles représentés par $R_7$ peuvent être substitués sur l'atome d'azote par un groupe alkyle inférieur ayant 1 à 4 atomes de carbone, et les groupes phényles et la partie phényle des groupes phénylalkyles inférieurs représentés par $R_7$ et/ou $R_8$ peuvent être substitués par un halogène ou un alkyle inférieur ayant 1 à 4 atomes de carbone;

la formule étant soumise aux conditions complémentaires suivantes:

(i) quand A est un groupe d'atomes requis pour former un anneau condensé [1,2-b]thiophène, Z est $—CH_2—CH_2$ ou $—CH{=}CH—$, p est 2, et soit:

1) B est un groupe d'atomes requis pour former un anneau benzène condensé, $R_2$ et $R_3$ sont tous deux l'hydrogène ou forment une double liaison, $R_4$ est l'hydrogène et $R_1$ est un alkyle $C_1—C_4$ ou $R_4$ et $R_1$ forment le second pont $(—CH_2—)_2$; ou

2) B est un anneau benzène X-substitué, $R_2$ et $R_3$ forment une double liaison, $R_4$ est l'hydrogène et $R_1$ est Me; alors $R_7$ ne peut être un groupe alkyle $C_1$—$C_4$ ou phényle (alkyle $C_1$—$C_4$);

(ii) quand A et B représentent respectivement un anneau benzène condensé et un benzène condensé ou un anneau benzène X-substitué, alors $R_1$ et $R_4$ forment ensemble un second pont $(—CH_2—)_2$ entre l'atome de carbone et l'atome d'azote auxquels ils sont fixés;

et, dans les définitions de Y ci-dessus, les groupes alkyles et alkényles possèdent jusqu'à 12 atomes de carbone, les groupes alkyles inférieurs (dans les groupes phénylalkyles inférieurs) possèdent 1 à 4 atomes de carbone, les groupes cycloalkyles possèdent 3 à 7 atomes de carbone et les groupes cycloalkylalkyles ont 4 à 12 atomes de carbone.

2. Composés suivant la revendication 1, caractérisés en ce que Z représente un groupe éthylène ou vinylène.

3. Composés suivant la revendication 1 ou 2, caractérisés en ce que $R_2$ et $R_3$ forment ensemble une seconde liaison.

4. Composés suivant n'importe laquelle des revendications 1 à 3, caractérisés en ce que $R_1$ et $R_4$ forment ensemble un pont de formule $—(CH_2)_2—$.

5. Composés suivant n'importe laquelle des revendications 1 à 4, caractérisés en ce que l'anneau défini par A est un anneau thiophène ou pyridine.

6. Composés suivant n'importe laquelle des revendications 1 à 5, caractérisés en ce que l'anneau défini par B est un anneau benzène condensé ou benzène X-substitué, X étant comme défini dans la revendication 1.

7. Composés suivant n'importe laquelle des revendications 1 à 6, caractérisés en ce qu'ils sont représentés par la formule générale II

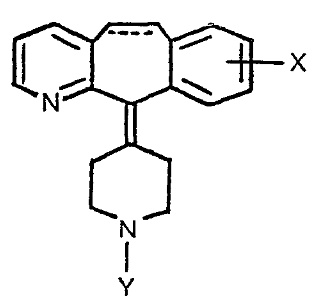

(II)

dans laquelle la ligne pointillée représente une double liaison facultative et X et Y sont comme définis dans la revendication 1.

8. Composés suivant n'importe laquelle des revendications 1 à 7, caractérisés en ce qu'il existe une simple liaison entre les positions 5 et 6.

9. Composés suivant n'importe laquelle des revendications 1 à 8, caractérisés en ce que X représente l'hydrogène, le chlore-8 et le brome-8.

10. Composés suivant n'importe laquelle des revendications 1 à 9, caractérisés en ce qu'ils sont
11-(N-carboéthoxy-4-pipéridylidène)-8-chloro-6,11-dihydro-5H-benzo[5,6]cyclohepta[1,2-b]pyridine;
11-(N-méthanesulfonyl-4-pipéridylidène)-6,11-dihydro-5H-benzo[5,6]cyclohepta[1,2-b]pyridine;
11-(N-carboéthoxy-4-pipéridylidène)-6,11-dihydro-5H-benzo[5,6]cyclohepta[1,2-b]pyridine;
11-(N-carbométhoxy-4-pipéridylidène)-6,11-dihydro-5H-benzo[5,6]cyclohepta[1,2-b]pyridine; ou
11-(N-carbophénoxy-4-pipéridylidène)-6,11-dihydro-5H-benzo[5,6]cyclohepta[1,2-b]pyridine.

11. Compositions pharmaceutiques caractérisées en ce qu'elles contiennent comme ingrédient actif un composé choisi parmi les composés de formule I suivant la revendication 1, et les composés exclus de la revendication 1 par les conditions (i) et (ii), en mélange avec un agent ou excipient pharmaceutiquement acceptable.

12. Procédé de fabrication de composés de formule I suivant la revendication 1, caractérisé en ce qu'un composé de formule générale III

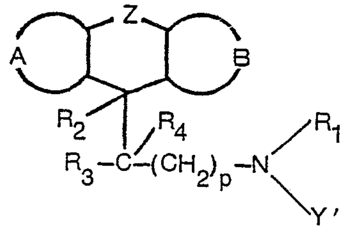

(III)

dans laquelle A, B, Z, $R_1$, $R_2$, $R_3$, $R_4$ et p sont comme définis dans la revendication 1, et Y' représente un groupe remplaçable, est soumis à réaction avec un composé de formule IV

$$TY \quad (IV)$$

dans laquelle Y est comme défini dans la revendication 1 et T représente un groupe pouvant être éliminé en même temps que Y', et, si on le désire, le composé de formule I ainsi obtenu peut être transformé en un autre composé de formule I par une transestérification du groupe Y.

13. Composés de formule I définis dans la revendication 1 et ceux exclus de la revendication 1 par les conditions (i) et (ii), caractérisés en ce qu'ils sont utilisés comme antihistaminiques.

**Revendications pour l'Etat contractant: AT**

1. Procédé de fabrication de composés de formule générale I

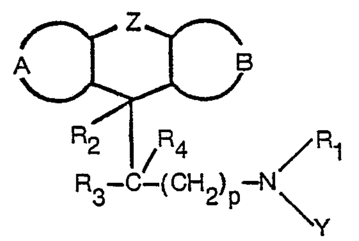

(I)

dans laquelle

A représente le groupe d'atomes requis pour former un anneau condensé aromatique hétérocyclique à 5 ou 6 membres ou un anneau benzène condensé;

B représente le groupe d'atomes requis pour former un anneau condensé pyridine, benzène ou benzène X-substitué dans lequel X est un halogène, avec la condition que B représente un anneau condensé benzène ou benzène X-substitué quand A représente un anneau hétérocyclique contenant de l'azote;

Z représente une liaison simple ou un groupe méthylène ou éthylène substitué ou non-substitué, le substituant étant un groupe oxo ou hydroxy, ou bien Z représente un vinylène;

$R_2$ représente l'hydrogène ou un groupe alkyle ayant 1 à 3 atomes de carbone;

$R_3$ représente l'hydrogène, ou

$R_2$ et $R_3$ forment ensemble une seconde liaison;

$R_4$ représente l'hydrogène;

$R_1$ représente un groupe alkyle ayant 1 à 6 atomes de carbone, ou

$R_1$ et $R_4$ forment ensemble un second pont $-(CH_2)_2-$ entre l'atome de carbone et l'atome d'azote auxquels ils sont fixés;

p représente 1 ou 2;

Y représente l'un des groupes $-COOR_7$ ou $-SO_2R_8$ dans lesquels

$R_7$ représente un groupe alkyle, cycloalkyle, cycloalkylalkyle, alkényle, phényle, phénylalkyle inférieur ou 2-, 3- ou 4 pipéridyle et

$R_8$ représente un groupe alkyle, cycloalkyle, cycloalkylalkyle, alkényle, phényle ou phénylalkyle inférieur, dans lequel les groupes alkyles, cycloalkyles, cycloalkylalkyles et alkényles représentés par $R_7$ peuvent être éventuellement substitués par le groupe

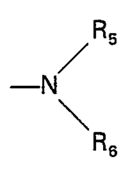

dans lequel $R_5$ et $R_6$ représentent chacun l'hydrogène ou un groupe alkyle ayant 1 à 6 atomes de carbone, les groupes pipéridyles représentés par $R_7$ peuvent être substitués sur l'atome d'azote par un groupe alkyle inférieur ayant 1 à 4 atomes de carbone, et les groupes phényles et la partie phényle des groupes phénylalkyles inférieurs représentés par $R_7$ et/ou $R_8$ peuvent être substitués par un halogène ou un alkyle inférieur ayant 1 à 4 atomes de carbone;

la formule étant soumise aux conditions complémentaires suivantes:

(i) quand A est un groupe d'atomes requis pour former un anneau condensé [1,2-b] thiophène, Z est $-CH_2-CH_2$ ou $-CH=CH-$, p est 2, et soit:

1) B est un groupe d'atomes requis pour former un anneau benzène condensé, $R_2$ et $R_3$ sont tous

deux l'hydrogène ou forment une double liaison, $R_4$ est l'hydrogène et $R_1$ est un alkyle $C_1$—$C_4$ ou $R_4$ et $R_1$ forment le second pont $(—CH_2—)_2$; ou

2) B est un anneau benzène X-substitué, $R_2$ et $R_3$ forment une double liaison, $R_4$ est l'hydrogène et $R_1$ est Me; alors $R_7$ ne peut être un groupe alkyle $C_1$—$C_4$ ou phényle (alkyle $C_1$—$C_4$);

(ii) quand A et B représentent respectivement un anneau benzène condensé et un benzène condensé ou un anneau benzène X-substitué, alors $R_1$ et $R_4$ forment ensemble un second pont $(—CH_2—)_2$ entre l'atome de carbone et l'atome d'azote auxquels ils sont fixés,

et, dans les définitions de Y ci-dessus, les groupes alkyles et alkényles possèdent jusqu'à 12 atomes de carbone, les groupes alkyles inférieurs (dans les groupes phénylalkyles inférieurs) possèdent 1 à 4 atomes de carbone, les groupes cycloalkyles possèdent 3 à 7 atomes de carbone et les groupes cycloalkylalkyles ont 4 à 12 atomes de carbone, caractérisé en ce qu'on fait réagir un composé de formule générale III

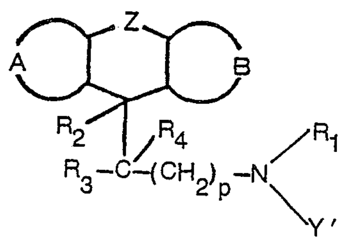

(III),

dans laquelle A, B, Z, $R_1$, $R_2$, $R_3$, $R_4$ et p sont comme définis dans la revendication 1, et Y' représente un groupe remplaçable, avec un composé de formule IV

TY (IV)

dans laquelle Y est comme défini dans la revendication 1 et T représente un groupe pouvant être éliminé en même temps que Y', et, si on le désire, le composé de formule I ainsi obtenu peut être transformé en un autre composé de formule I par une transestérification du groupe Y.

2. Procédé suivant la revendication 1, caractérisé en ce qu'on fait réagir un composé de formule III dans laquelle Y' est méthyle, avec un composé de formule

$$T\ COOR_7$$

dans laquelle T est un halogène, de préférence le chlore, et $R_7$ est comme défini dans la revendication 1.

3. Procédé suivant la revendication 1, caractérisé en ce qu'on fait réagir un composé de formule générale III dans laquelle Y' est l'hydrogène, avec un composé de formule

$$T\ COOR_7$$

dans laquelle T représente le groupe $OR_7$, $R_7$ étant comme défini dans la revendication 1.

4. Procédé suivant la revendication 1, caractérisé en ce qu'on fait réagir un composé de formule III dans laquelle Y' représente l'hydrogène, avec un composé de formule IV dans laquelle T représente un halogène, de préférence le chlore, et Y représente le groupe $SO_2R_8$, $R_8$ étant comme défini dans la revendication 1.

5. Procédé suivant n'importe laquelle des revendications 1 à 4, caractérisé en ce qu'on utilise comme composé de départ, un composé de formule II'

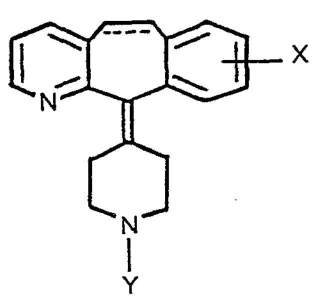

(II')

dans laquelle X est comme défini dans la revendication 1 et Y' est comme défini comme dans n'importe laquelle des revendications 1 à 4.

6. Procédé suivant la revendication 5, caractérisé en ce qu'on fait réagir un composé de formule II' dans laquelle X représente l'hydrogène ou le chlore-8, avec un composé de formule

$$TCOOC_2H_5$$

Y' et T étant comme définis dans n'importe laquelle des revendications 1 à 4.

7. Procédé de fabrication d'une composition pharmaceutique, caractérisé en ce qu'on mélange un composé choisi parmi les composés de formule 1 comme définis dans la revendication 1 et ceux exclus de la revendication 1 par les conditions (i) et (ii), avec un agent ou excipient pharmaceutiquement acceptable.